# EUROPEAN PATENT SPECIFICATION

(11) **EP 2 531 834 B1**
(45) Date of publication and mention of the grant of the patent: **22.07.2020**
(21) Application number: 11703497.5
(22) Date of filing: 20.01.2011
(51) Int. Cl.: G01N 21/27, G01N 33/96, A61M 1/02

(54) **METHOD FOR DETERMINING THE RELIABILITY OF A DEVICE FOR MEASURING THE CONCENTRATION OF A SUBSTANCE IN WHOLE BLOOD**
VERFAHREN ZUR BESTIMMUNG DER VERLÄSSLICHKEIT EINER VORRICHTUNG ZUR MESSUNG DER KONZENTRATION EINES STOFFES IN VOLLBLUT
PROCÉDÉ DE DÉTERMINATION DE LA FIABILITÉ D'UN DISPOSITIF DESTINÉ À MESURER LA CONCENTRATION D'UNE SUBSTANCE DANS DU SANG TOTAL

(30) Priority: 04.02.2010 US 301457 P; 01.02.2010 NL 1037672
(43) Date of publication of application: 12.12.2012
(73) Proprietor: Eurotrol B.V., 6716 BS Ede (NL)
(72) Inventor: JUNGERIUS, Bart Johan, NL-6716 BS EDE (NL); HUIZING, Carolina Johanna, NL-6716 BS EDE (NL); MAAS, Bartholomeus Henricus Antonius, NL-6716 BS EDE (NL)
(74) Representative: Nederlandsch Octrooibureau
(86) International application number: PCT/NL2011/050034
(87) International publication number: WO 2011/093697

(56) References cited:
- WO-A1-03/090839
- US-A- 4 731 330
- HUIZING C J ET AL: "GlucoTrol-WB: A highly commutable whole blood quality control for glucose point-of-care testing", POINT OF CARE, LIPPINCOTT WILLIAMS & WILKINS, US, vol. 29, no. 3, 1 September 2010 (2010-09-01), page 129, XP008128455, ISSN: 1533-029X

## Description

The present invention relates to a method for determining the reliability of a device for measuring the concentration of a substance in whole blood, and the disclosure relates to a method for treating whole blood. The disclosure also relates to a container and to a kit.

The invention is in the field of measuring the concentration of a substance in whole blood. Within the framework of the present invention, the term "whole blood" is understood to mean blood from which no components have been removed. In order to make the blood non-coagulable, an anticoagulant and possibly an additive, such as an antibiotic, for example, may be added. The whole blood is obtained from a mammal, which term includes humans, cows, horses or pigs.

As will be explained hereinafter, the substance is in principle any compound or substance that occurs in whole blood, the concentration of which is to be measured (also referred to as an "analyte"). Said substance may occur naturally, for example in the case of metabolites, such as glucose, and enzymes, or be present in the blood as a result of external influences (for example viral and bacterial antigens in the case of infections, ingested medicines and addictive drugs). A common property of the substances within the framework of the present invention is that for various reasons (such as ingestion and/or digestion by cells or enzymes present in the blood) their concentration does not remain constant in whole blood, in other words, is not "stable" in said whole blood, whereas it is stable in plasma or serum separated from blood.

It has become common practice in current human as well as veterinary medical practice to measure the concentration of a substance in whole blood at locations other than a laboratory. The substance is measured in whole blood, because it is not possible at the so-called "point of care" (for example the location of an accident, a stable or cowhouse or sty, the emergency department of a hospital, a doctor's surgery, an operating room, or a private home in the case of measuring devices for home use) to separate plasma and measure the concentration of the substance by means of the devices that are commonly used in a laboratory. Usually, mobile and or hand-held devices are used for measuring the concentration of a substance on site or at a point of care ("point-of-care-testing"). Just like measuring devices for laboratory use, which are generally used for measuring substances in blood plasma, also devices for measuring the concentration of a substance in whole blood need to be subjected to reliability measurements.

The term "reliability" as used within the frame of the present invention comprises the reliability and/or the precision of the measuring results, the correctness of the measuring results and possibly other characteristics for determining the quality of the device. In a medical context, this determination is used within a quality assessment programme. What is and what is not considered to be reliable depends on the nature of the substance in question, of course, and may be imposed by the relevant legislation and rules. For a complete quality assessment, additional laboratory measurements are in many cases needed for measuring the concentration of the substance that is inherently present in the control blood, in particular in the case of a natural substance.

A control sample containing a known amount of a substance is needed for determining the reliability of a device for measuring the concentration of said substance in whole blood. It stands to reason that the sample must be comparable as much as possible to the material that will be used for the measurements in practice. Consequently, the control sample must be based on whole blood functioning as control blood, within the framework of the present invention also referred to as whole control blood. The whole control blood is not necessarily obtained from only one organism or donor. Since according to the invention, as mentioned above, the concentration of the substance does not remain constant in whole blood, the problem arises that the reliability cannot be satisfactorily determined because the amount of the substance in the control sample based on whole control blood changes as a function of time. In addition, whole blood will only keep for a limited, in any case not prolonged, period of time. Haemolysis occurs, among other things, as a result of which haemoglobin is released, for example. The consequence of all this is, of course, that the result of the measurement in the whole control blood is uncertain, so that it is not possible to determine the reliability on the basis thereof when pre-prepared samples of whole control blood are used. It is noted that on-site mixing of whole control blood with the substance to be measured at the time of the measurement is laborious and undesirable, and in any case not practical, for the intended application. Moreover, on-site mixing would be too inaccurate.

Prior art attempts have been made to solve the above problem by proposing methods in which use is made of whole control blood that has been treated prior to the control measurement. As will be summarised below, the known pretreatments lead to new problems, however, so that it is still not possible to determine the reliability in a satisfactory manner. For the sake of completeness it may furthermore be noted that it has even been proposed to prepare an aqueous control sample. As the "matrix" is completely different from that of whole blood in that case, erratic results are obtained when using an aqueous control sample, and some devices designed for whole blood measurements even generate an error message. The pre-treatments of the whole control blood proposed in the prior art include: (i) freeze-drying, using additives; increasing the stability of the cell membranes; concentrating red blood cells with the addition of preservatives; inhibiting the haemolysis, using inhibitors; maintaining or increasing the 2,3-diphosphoglycerate concentration, using additives; chemically fixing blood cells, or (ii) inhibiting processes that would lead to an increased or decreased concentration of the substance to be measured in whole blood, using inhibitors or acidifiers. Said pretreatments are not satisfactory at all, because they lead to new problems: in the treatments mentioned under (i), use is made of additives, preservatives, inhibitors and/or fixing compounds which influence the measurement, since they concern saccharides, for example, or are toxic and change the structure of the membranes in an unnatural manner; in the treatments mentioned under (ii), agents are used which cause haemolysis, which undesirably influence the measurement, and which in many cases inhibit the processes in question only temporarily.

Several publications are known.

US 4731330 (A) recites a whole blood control sample and a method of preparing the sample are disclosed. The method comprises collecting a whole blood sample from one or more donor, separating each sample into red blood cells and plasma, fixing the red blood cells, mixing the fixed red blood cells with plasma from the same or a different donor to produce a suspension, quick-freezing the suspension before the red blood cells can settle, and lyophilizing the frozen suspension. The control sample therefore comprises a lyophilized mixture of fixed red blood cells and plasma solids.

US 4731330 (A) recites (chemically) fixing red blood cells, which is undesirable, for instance because properties of the sample are changed, and as a consequence the reproducibility of a subsequent measurement is affected. Furthermore, fixing involves an extra step.

Even further, water is removed from the sample. Cells and plasma remain together. This involves problems, for instance as mentioned above relating to the prior art.

Water is removed from the sample and then the sample is frozen. This again involves an extra step and may change properties of the sample, jeopardizing the reproducibility, for example.

Water is only added shortly before measurement.

WO 03090839 (A1) recites an apparatus and methods to consistently separate and concentrate selected blood components. The system includes, e.g., a computerized fluid handling system to transfer blood components between a centrifugal blood separation disc, containers and a concentrator. The above document does not recite a cell free fraction. Possibly some components present in whole blood can be separated into a further fraction, but this is regarded as a marginal adjustment to whole blood. As such characteristics of whole blood do not change significantly and problems of the prior art are still not solved, e.g. measurements are still not reproducible.

It has surprisingly been found that the aforesaid pre-treatments of whole control blood are not necessary and that as a result the addition of agents that affect the measurement adversely and in any case undesirably can be prevented by providing a method for determining the reliability of a device for measuring the concentration of a substance in whole blood, wherein, as a first step, a separated, stored, cell-free blood fraction, selected from plasma and serum, and a suspension of blood cells, each separated from whole control blood, are mixed together. Prior to said mixing, a known amount of the substance is added to the cell-free blood fraction. Said mixing results in reconstituted whole control blood enriched with said substance. As mentioned above, the whole control blood as such is not necessarily obtained from only one organism or donor.

The method according to the present invention is characterised by the following successive steps: a) mixing a separated, stored, cell-free blood fraction, selected from plasma and serum, and a suspension of blood cells, each separated from whole control blood, with a known amount of the substance being added to the cell-free blood fraction, wherein said cell-free blood fraction and said suspension of blood cells are stored in a container that comprises at least two connected components, preferably at a temperature of 2-8° C; b) measuring, by means of the device, the concentration of the substance in the reconstituted whole control blood enriched with said substance in step a); c) determining the reliability of the device on the basis of the difference between (i) the known concentration of the substance after step a) and (ii) the result of the measurement of the measurement of step b). A method according to the present disclosure is characterised by the following successive steps: a) mixing a separated, stored, cell-free blood fraction, selected from plasma and serum, and a suspension of blood cells, each separated from whole control blood, with a known amount of the substance being added to the cell-free blood fraction; b) measuring, by means of the device, the concentration of the substance in the reconstituted whole control blood enriched with said substance in step a); c) determining the reliability of the device on the basis of the difference between (i) the known concentration of the substance after step a) and (ii) the result of the measurement of the measurement of step b).

In practice, the results of such a method can also be compared for each type of device, with "outliers" being regarded as suspect. This is also referred to as "peer grouping" in the art.

If the substance in question already naturally occurs in a specific concentration in the whole control blood, said concentration will of course have to be measured separately (for example previously in a laboratory) in order to obtain in step (c) the "known concentration of the substance after step (a)". If the substance is glucose, for example, the "known concentration of the substance after step (a)" is the sum of the separately measured natural concentration of glucose and the concentration that results from the addition of "the known amount" of glucose to a known amount of cell-free blood fraction of the whole control blood.

The main advantage of the method according to the invention is the fact that the concentration of the substance to be measured will remain stable in the cell-free blood fraction for a prolonged period of time of a few weeks to a few months. It has furthermore been found that, following the reconstitution of a suspension of blood cells with the cell-free blood fraction for the purpose of obtaining a sample of substance-enriched whole control blood, any change(s) that may have occurred in the suspension of blood cells, for example a negligible increase of the free haemoglobin content, apparently do (does) not affect the measurement. The separate storage of cell free blood fraction and suspension of blood cells is a temporary measure, which can be reversed without any problem. Since the substance has been added to the cell-free suspension, the blood cells cannot influence the concentration of the substance.

Said storage can in principle take place at room temperature or even at 37 °C. It is to be preferred, however, to store the cell-free blood fraction and the suspension of blood cells at a temperature of 2-8 °C. Because of said low temperature, various metabolic processes of the blood cells are strongly retarded and even stopped, so that practically no "natural" processes that affect the concentration of the substance will take place. Thus, the formation of methaemoglobin (MetHb) and haemolysis, for example, are retarded.

Preferably, the cell-free blood fraction and the suspension of blood cells to be used in step (a) are obtained by centrifuging whole control blood and removing 5-90 volume per cent of the cell-free blood fraction so as to obtain a suspension of blood cells, possible using one or more additives. In general the same or a different additive can be added to the whole blood, to the cell-free blood fraction or to the suspension of blood cells. To obtain plasma, a commonly used anticoagulant, such as 3.98 % trisodium citrate-dihydrate and 0.016 % citric acid in water, for example, can be added to whole blood. The additive may also be an and die biotic, for example gentamycin sulphate. Preferably, 75-85 volume per cent of the cell-free blood fraction is removed. Said percentage depends on the animal species from which the whole control blood has been obtained. The intention is to have the haematocrit content of the substance-enrichted reconstituted whole control blood correspond to the haematocrit content of the donor's or patient's whole blood, the concentration of the substance in which blood is to be measured by means of the device at the point of care. The whole blood may be bovine blood, for example, whilst a human's whole blood is to be tested at the point of care (POC). Preferably, the haematocrit content of the suspension of blood cells is adjusted to 20-90. Usually, this is done by adding or removing cell-free blood fraction. It is in particular preferable to use a haematocrit content of 50-80 volume per cent. Said volume percentages depend on the desired haematocrit content after the cell-free blood fraction and the suspension of blood cells have been mixed together, and thus, for the aforesaid reason, also on the animal species.

The cell-free blood fraction may be subjected to one or more purification steps that are known as such in the art: for example, freezing and subsequently thawing the cell-free blood fraction, or acidifying and subsequently neutralising the cell-free blood fraction.

According to the invention, the whole control blood consists of mammalian blood, including human blood. Preferably, the blood is bovine blood or human blood. If the devices are to be used for measurements in whole human blood, it is of course preferable to use human control blood. Human blood products are generally expensive and difficult to obtain in large volumes, whilst constituting a risk as regards infectious diseases (for example AIDS or hepatitis C). Bovine blood appears to be a good substitute for human blood. Bovine blood, however, has a lower haematocrit content than human blood, so that bovine blood intended for use as whole control blood needs to be "thickened" in order to make it more comparable to human blood.

In principle, the suspension of blood cells from the whole control blood and the substance-enriched cell-free blood fraction can be stored in any suitable container. For practical reasons, for example for easier mixing in the container, either the suspension of blood cells from the control blood or the substance-enriched cell-free blood fraction can be stored in a container that comprises at least two components.

Preferably, the substance is added to the cell-free blood fraction in an amount such that a desired concentration of the substance in the sample is obtained after reconstitution of the cell-free blood fraction with an amount of the suspension of blood cells into a sample of substance-enriched whole control blood. To measure a range of concentrations, for example for the purpose of drawing up a reliability curve or determining the reliability in the case of deviating concentrations, it is preferable to add different concentrations of the substance to different samples of the cell-free blood fraction.

The substance-enriched cell-free blood fraction or the suspension of blood cells, as the case may be, is preferably aseptically distributed over sterile bottles. The term "bottles" as used within the framework of the present invention is to be broadly interpreted, they may for example be vessels or recipients of hard or soft plastic or glass. Preferably, the aforesaid container is connected to a bottle containing the substance-enriched cell-free blood fraction or the suspension of blood cells prior to the mixing step.

According to the present invention, the substance as generally described above is selected from the group consisting of metabolites, tracers for infectious diseases, toxicologically relevant substances and tracers therefor.

Examples of metabolites are: glucose, lactate, lipids and ketones (such as beta-hydroxybutyric acid).

Examples of tracers for infectious diseases are: Streptococcus A and B, HIV, Haemophilus influenza and RSV.

Examples of toxicologically relevant substances are: alcohol, addictive drugs, doping and medicines.

In many circumstances it is essential that the concentration of glucose in whole blood be determined quickly and in a reliable manner, for example in order to find whether the subject in question has diabetes. A significant amount of interaction takes place also between glucose and red blood cells, which interaction is described in the prior art. For these reasons glucose is preferably used as the substance to be measured in the present method.

The present disclosure also relates to a method for treating whole blood.

As explained in the foregoing, there is a need for a method for providing whole blood which can be used in obtaining a control sample on the basis of whole blood. The whole blood can for example be used for determining the reliability of a device for measuring the concentration of a substance in whole blood.

The method according to the present disclosure for treating whole blood comprises the following steps to be successively carried out: (a) centrifuging whole blood, with the possible use of one or more additives, so as to obtain a cell-free blood fraction selected from plasma or serum; (b) removing 5-90 volume per cent of the cell-free blood fraction, with the possible use of one or more additives, so as to obtain a suspension of blood cells; (c) adding such an amount of a substance to be measured to the cell-free blood fraction that a desired concentration of the substance in the sample is obtained following the reconstitution into a whole blood sample of (i) the substance-enriched cell-free blood fraction and (ii) an amount of the suspension of blood cells; (d) storing the substance-enriched cell-free blood fraction and the suspension of blood cells separately from each other.

Preferably, 75-85 volume per cent of the cell-free blood fraction is removed in step (b). The haematocrit content of the suspension of cells is preferably set at 20-90 volume per cent, in particular at 50-80 volume per cent.

An improved storage life can be obtained if the cell-free blood fraction is also subjected to one or more purification steps, which are known as such in the art: the cell-free blood fraction can frozen and subsequently thawed, or be acidified and subsequently neutralized.

For the above reasons, the cell-free blood fraction and the suspension of blood cells are preferably stored at a temperature of 2-8 °C.

According to the disclosure, the blood consists of mammalian blood, including human blood. Preferably, the blood is bovine blood or human blood.

The storage of either the suspension of blood cells of the blood, or of the substance-enriched so-free blood fraction in a holder which comprises at least two components is very suitable, for example for the purpose of facilitating the mixing step. The suspension or the cell-free blood fraction containing the substance is preferably aseptically stored in sterile bottles. In order to facilitate the practical application of the method it is preferable if a container containing either the suspension of blood cells or the substance-enriched cell-free blood fraction is connected to a bottle containing the substance-enriched so-free blood fraction or the suspension of blood cells.

According to the invention, the substance is selected from the group consisting of metabolites, tracers for infectious diseases, toxicologically relevant substances and tracers therefor. Examples of substances are listed above. For the above reason, the substance is preferably glucose.

The present disclosure also relates to a container which contains either a suspension of blood cells obtained from whole blood or a cell-free blood fraction enriched with the substance to be measured, which cell-free blood fraction or which suspension of blood cells has been obtained by using the method according to the invention.

The disclosed container is preferably an assembly consisting of a dosing part and a supply part, wherein the dosing part contains either a suspension comprising blood cells obtained from whole blood or a cell-free blood fraction enriched with a substance to be measured, and wherein the metering part and the supply part are configured to cooperate with one another, such that a pressure chamber having a changeable volume is formed when the supply part and the dosing part are joined together, which pressure chamber functions to obtain a pumping action for mixing the suspension of blood cells with the cell-free blood fraction to be added or mixing the cell-free blood fraction with the suspension of blood cells to be added, as the case may be, which suspension and which cell-free blood fraction have been obtained by using a method as described in the foregoing. A very suitable container is described in WO 2007/040396.

In a special embodiment of the disclosed container, a bottle containing either the suspension of blood cells obtained from whole blood or the cell-free blood fraction obtained from whole blood, which is enriched with an added, known amount of the substance, is connected to the container or to the supply part of a container comprising a supply part and a dosing part. Bottles that can be used for this purpose are described in the foregoing.

Finally, the disclosure relates to a kit for determining the reliability of a device for measuring the concentration of a substance in whole blood, which kit comprises (i) at least one container containing either a suspension of blood cells of whole control blood or a cell-free blood fraction enriched with a substance to be measured, and (ii) at least one bottle containing cell-free blood fraction comprising a known concentration of the substance or the suspension of blood cells, and (iii) possibly instructions for mixing the cell-free blood fraction with the suspension of blood cells, in order to obtain one or more whole control blood samples, which suspension and which cell-free blood fraction have been obtained by using a method as described in the foregoing, and for measuring the concentration of the substance in the sample or the samples by means of the device for measuring the concentration of the substance in whole blood whose reliability is to be determined.

The invention will now be explained by means of examples.

The following is presented in the associated drawings.

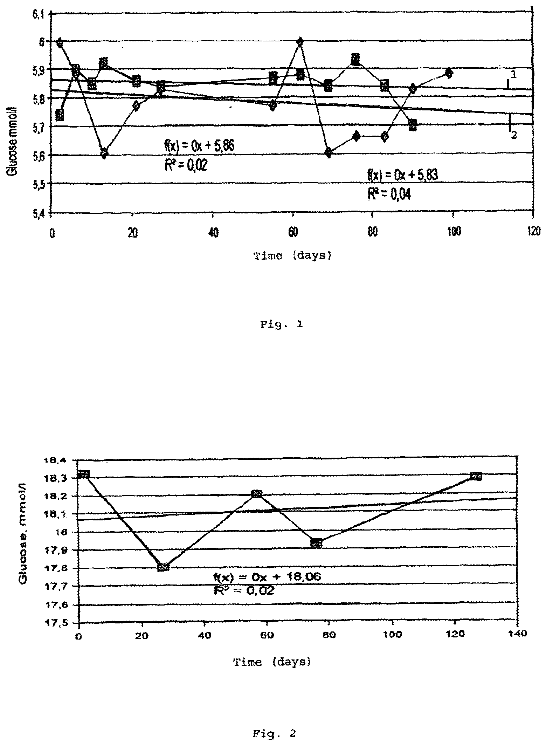

Figure 1: The glucose concentration of samples of reconstituted whole control blood was measured as a function of time, using an YSI 2300 STAT Plus (black square) and a B-glucose analyser (HemoCue, Sweden) (black diamond). Ordinate: glucose concentration in mmol/l; absis: time in days: 1: linear regression for the YSI 2300 STAT Plus (f(x) = Ox + 5.86; R2 = 0.02); 2: linear regression for the B-glucose analyser (f(x) = Ox + 5.83; R2 = 0.04). The experiment is described in Example 4.
Figure 2: The glucose concentration of samples of reconstituted whole control blood was measured, using an YSI 2300 STAT Plus device (black square). The straight line represents the linear regression (f(x) = Ox + 18.06; R2 = 0.02). The experiment is described in Example 4.

### Example 1.

The present example is concerned with obtaining and treating bovine whole control blood and to obtaining separated stored plasma (with glucose added thereto) and a suspension of blood cells.

Blood was drawn from the vena jugularis of a cow and collected in glass infusion bottles, to which 100 ml of an anticoagulant (3.98% trisodiumcitrate dihydrate and 0.016% citric acid in distilled water) had been added per litre of blood. Per litre of blood, 0.8 ml of a solution of 50 mg/ml of antibiotic gentamycin sulphate was added.

The haematocrit content of the blood was determined in heparinized capillaries, which where centrifuged in a haematocrit centrifuge (Sigma 1-15P, 5 minutes, 12,000 g). The haematocrit content was 28%. The blood was transferred to transfusion bags and centrifuged (Heraes Sepatech, 15 minutes, 3000 rpm). Using a plasma extractor (Fenway FDR 4414), 75% of the plasma was removed. The plasma was collected in a beaker. To remove the remaining red blood cells from the plasma, the plasma was distributed over centrifuge bottles and centrifuged (Sorvall RC SB Plus, 10 minutes, 9000 rpm). Following this, the plasma was transferred to a beaker.

The suspension of blood cells obtained by removing plasma was transferred from the transfusion bags to a beaker of plastic material. The haematocrit content of the suspension was determined, in the manner described above, to be 75%; using plasma, this percentage was reduced to 60%. The suspension of blood cells was distributed over containers comprising two compartments, in particular ACU-CAPs (brand), available from Eurotrol B.V., Ede, Netherlands, in an amount of 2 ml of suspension per ACU-CAP. In order to keep the suspension of cells homogeneous during the distribution process, the blood was continuously agitated, using a magnetic agitator, while being distributed.

The glucose content of the plasma was measured by means of a B-glucose analyser (HemoCue, Sweden). Glucose (D-(+)-Glucose) was added in an amount such that the concentration thereof in the plasma was three times higher than the desired concentration in the reconstituted whole control blood: the desired glucose content in the reconstituted blood was 6 mmol/l, the glucose concentration in the plasma was 18 mmol/l.

The plasma was filtered through a sterile filter and collected in a sterile glass bottle, whereupon the plasma was distributed over sterile bottles, 1 ml of plasma per bottle. The bottle was sealed with a sterile rubber stopper. The supply part of each ACU-CAP was connected to a glass bottle (containing plasma and glucose). The ACE-CAPs with the bottles connected thereto were stored at 4° C.

After mixing of the suspension of blood cells with the plasma, a sample of the glucose-enriched bovine whole control blood was obtained, which sample was suitable for determining the reliability of a device for measuring glucose in human whole blood.

Comparable results were obtained when cell-free blood fraction serum was used instead of plasma.

### Example 2.

The present example is concerned with obtaining and treating human whole control blood and to obtaining separated stored plasma (with glucose added thereto) and a suspension of blood cells.

Blood was drawn from human donors and collected in transfusion bags, to which 800 mg of trisodium-EDTA in 20 ml of distilled water had been added as an anticoagulant. Per litre of blood, 0.8 ml of a solution of 50 mg/ml of antibiotic gentamycin sulphate was added.

The haematocrit content of the blood was determined in heparinized capillaries, which where centrifuged in a haematocrit centrifuge (Sigma 1-15P, 5 minutes, 12,000 g). The haematocrit content was 36%. The blood was transferred to transfusion bags again and centrifuged (Heraes Sepatech, 15 minutes, 3000 rpm). Using a plasma extractor (Fenway FDR 4414), 78% of the plasma was removed. The plasma was collected in a beaker. To remove the remaining red blood cells from the plasma, the plasma was distributed over centrifuge bottles and centrifuged (Sorvall RC SB Plus, 10 minutes, 9000 rpm). Following this, the plasma was transferred to a beaker.

The suspension of blood cells obtained by removing plasma was transferred from the transfusion bags to a beaker of plastic material. The haematocrit content of the suspension was determined, in the manner described above, to be 73%; using plasma, this percentage was reduced to 60%. The suspension of blood cells was distributed over containers comprising two compartments, in particular ACU-CAPs (brand), available from Eurotrol B.V., Ede, Netherlands, in an amount of 2 ml of suspension per ACU-CAP. In order to keep the suspension of cells homogeneous during the distribution process, the blood was continuously agitated, using a magnetic agitator, while being distributed.

The glucose content of the plasma was measured by means of a B-glucose analyser (HemoCue, Sweden): it was determined to be 3.5 mmol/l. Glucose (D-(+)-Glucose) was added in an amount such that the concentration thereof in the plasma was 18 mmol/l.

The plasma was filtered through a sterile filter and collected in a sterile glass bottle, whereupon the plasma was distributed over sterile bottles, 1 ml of plasma per bottle. The bottle was sealed with a sterile rubber stopper. The supply part of each ACU-CAP was connected to a glass bottle (containing plasma and glucose). The ACU-CAPs with the bottles connected thereto were stored at 4 °C. Comparable results were obtained when cell-free blood fraction serum was used instead of plasma.

### Example 3.

Different devices for measuring the concentration in whole blood were compared with each other, using the methods according to the present invention; in particular as described in Example 1. The ACU-CAPs connected to bottles of plasma were heated to room temperature for 10 minutes. Then the cells were carefully mixed with the plasma by transferring the contents of the ACU-CAP back and forth between the ACU-CAP and the bottle three times. The dosing part (CAP) of the ACU-CAP was removed and the whole blood sample was drawn from the bottle by means of a syringe. From said syringe, the glucose concentration of the sample was measured, using different devices: two different point-of-care devices (X and Y) and one "classic" laboratory device (Z) while duly observing the producer's instructions in each case. The obtained results are summarized in Table 1.

**Table 1.**

| Measuring device | Average of 6 measurements | Standard deviation |
|---|---|---|
| X | 6.5 | 0.23 |
| Y | 7.3 | 0.23 |
| Z | 5.78 | 0.06 |

The measuring results obtained using POC devices were compared with the measuring result obtained using the laboratory device. The extent to which deviations are or are not admissible depends on the relevant legislation and rules and on standards that are usual in the art.

### Example 4.

The present example is concerned with the stability of the glucose concentration in reconstituted whole control blood and in glucose-enriched plasma of bovine whole control blood obtained and treated as described in Example 1. The present example is also concerned with the stability of whole blood based on the measured concentrations of free haemoglobin, methaemoglobin (MetHb) and oxyhaemoglobin (O₂Hb) and on the basis of the pH-values.

The glucose concentration of reconstituted samples was measured
as a function of time, using a YSI 2300 STAT Plus device (black square) and a B-glucose analyzer (black diamond) (HemoCue, Sweden) (see Figure 1). After 90 days the glucose concentration in the samples was at a level comparable to that on day 0. The linear regression (1: YSI 2300 SMT; 2: B-glucose analyser) was practically horizontal; from the R2-values (0.02 and 0.04, respectively) it follows that no significant connection exists between the glucose concentration and time.

From measurements in the plasma of the reconstituted whole blood samples (obtained by centrifuging respective amounts of 1 ml of reconstituted whole blood at 13,000 rpm in a MicroCentour centrifuge for 10 minutes - and determining the haemoglobin content of the obtained plasma by means of a Plasma/Low Hb analyser (HemoCue, Sweden) it appeared that the concentration of free haemoglobin exhibited a substantially linear connection with time, and that the free haemoglobin content was 2.5 g/l after 90 days. The increase of the free haemoglobin content to 2.5 g/l over a period of 90 days and any other changes in the blood cells apparently did not affect the results of the glucose measurements. By way of comparison it is noted that the concentrations of free haemoglobin in human blood plasma to which, according to the prior art, sodium iodine acetate had been added as a glycolysis inhibitor, was 12-15 gl/l after 7 days already.

The glucose content in samples of glucose-enriched plasma (obtained in the manner described in Example 1 and stored at 5° C) was measured using a YSI 2300 STAT Plus device. From figure 2 it appears that no changes occurred in the glucose concentration for a period of at least 120 days (the R2 value was 0.02).

It is a known fact that the MetHb content increases and the O2Hb content strongly decreases when whole blood ages. Consequently, the percentages of said haemoglobin variants of the aforesaid whole control blood samples were measured as a function of time. The haemoglobin fractions were measured by means of a Siemens Rapidlab 865. The MetHb percentage varied from 0.1% to 0.5% during a period of 90 days, and the O2Hb percentage varied from 98.4% to 99.6%. This minimal change showed that the blood cells in question did not exhibit any signs of ageing for at least 90 days.

One of the signs of ageing is acidification. Using a Siemens Rapidlab 865, the pH-values of reconstituted samples were measured as a function of time. The pH-values remained at a physiologically normal level of about 7.4 for at least 90 days. This is important also because a possible decrease of the pH-values may lead to haemolysis (which is undesirable).

## Claims

1. A method for determining the reliability of a device for measuring the concentration of a substance in whole blood, comprising the following successive steps:
a) mixing a separated, stored, cell-free blood fraction, selected from plasma and serum, and a suspension of blood cells, each separated from whole control blood, with a known amount of said substance being added to said cell-free blood fraction, wherein said cell-free blood fraction and said suspension of blood cells are stored in a container that comprises at least two connected components, preferably at a temperature of 2-8° C;
b) measuring, by means of said device, the concentration of said substance in the reconstituted whole control blood enriched with said substance in step a);
c) determining the reliability of said device on the basis of the difference between (i) the known concentration of said substance after step a) and (ii) the result of the measurement of step b).

2. A method according to claim 1, **characterised in that** said cell-free blood fraction and said suspension of blood cells to be used in step (a) are obtained by centrifuging whole control blood and removing 5-90 volume per cent of the cell-free blood fraction so as to obtain a suspension of blood cells, possible using one or more additives, wherein preferably 75-85 volume per cent of the cell-free blood fraction is removed.

3. A method according to claim 2, **characterised in that** the haematocrit content of the suspension of blood cells is adjusted to 20-90, wherein preferably the haematocrit content is adjusted to 50-80 volume per cent.

4. A method according to any one of claims 1-3, **characterised in that** said whole control blood consists of mammalian blood, including human blood, preferably said blood is bovine blood or human blood.

5. A method according to any one of claims 1-4, **characterised in that** said substance-enriched cell-free blood fraction is aseptically stored in sterile bottles.

6. A method according to any one of claims 1-5, **characterised in that** said substance is added to said cell-free blood fraction in an amount such that a desired concentration of said substance in a sample is obtained after reconstitution of said cell-free blood fraction with an amount of said suspension of blood cells into a sample of whole control blood enriched with said substance, wherein preferably different concentrations of the substances are added to different samples of said cell-free blood fraction.

7. A method according to any one of claims 1-6, **characterised in that** said substance is selected from a group consisting of metabolites, tracers for infectious diseases, toxicologically relevant substances and tracers therefore, preferably said substance is glucose.

## Patentansprüche

1. Verfahren zur Bestimmung der Zuverlässigkeit einer Vorrichtung zum Messen der Konzentration einer Substanz in Vollblut, welches die folgenden aufeinanderfolgenden Schritte aufweist:
a) Mischen einer separierten, gelagerten, zellfreien Blutfraktion, ausgewählt aus Plasma und Serum, und einer Suspension von Blutzellen, die jeweils vom Kontroll-Vollblut separiert sind, wobei eine bekannte Menge der Substanz zu der zellfreien Blutfraktion hinzugefügt ist; wobei die zellfreie Blutfraktion und die Suspension der Blutzellen in einem Behälter, der mindestens zwei verbundene Komponenten aufweist, gelagert wird, und zwar vorzugsweise bei einer Temperatur von 2 bis 8°C;
b) Messen der Konzentration der Substanz in dem mit der Substanz angereicherten rekonstituierten Kontroll-Vollblut in Schritt a) mittels der Vorrichtung;
c) Bestimmen der Zuverlässigkeit der Vorrichtung auf der Basis der Differenz zwischen (i) der bekannten Konzentration der Substanz nach Schritt a) und (ii) dem Resultat der Messung von Schritt b).

2. Verfahren nach Anspruch 1, **dadurch gekennzeichnet, dass** die zellfreie Blutfraktion und die Suspension von Blutzellen. die in Schritt (a) verwendet werden sollen, durch Zentrifugieren von Kontroll-Vollblut und durch Entfernen von 5 - 90 Volumenprozent der zellfreien Blutfraktion erhalten werden, um eine Suspension von Blutzellen zu erhalten, möglicherweise unter Verwendung von einem oder mehreren Zusatzstoffen, wobei vorzugsweise 75 - 85 Volumenprozent der zellfreien Blutfraktion entfernt werden.

3. Verfahren nach Anspruch 2, **dadurch gekennzeichnet, dass** der Hämatokritgehalt der Suspension von Blutzellen auf 20-90 eingestellt wird, wobei der Hämatokritgehalt vorzugsweise auf 50-80 eingestellt wird, und zwar in Volumenprozent.

4. Verfahren nach einem der Ansprüche 1 - 3, **dadurch gekennzeichnet, dass** das Kontroll-Vollblut aus Säugetierblut, einschließlich menschlichem Blut besteht, wobei das Blut vorzugsweise Rinderblut oder menschliches Blut ist.

5. Verfahren nach einem der Ansprüche 1 - 4, **dadurch gekennzeichnet, dass** die substanzangereicherte zellfreie Blutfraktion aseptisch in sterilen Flaschen gelagert wird.

6. Verfahren nach einem der Ansprüche 1 - 5, **dadurch gekennzeichnet, dass** die Substanz zu der zellfreien Blutfraktion in einer solchen Menge hinzugefügt wird, dass eine gewünschte Konzentration der Substanz in einer Probe erhalten wird nach der Rekonstitution der zellfreien Blutfraktion mit einer Menge der Suspension von Blutzellen in eine Probe von Kontroll-Vollblut, welches mit der Substanz angereichert ist, wobei vorzugsweise unterschiedliche Konzentrationen der Substanzen zu verschiedenen Proben der zellfreien Blutfraktion hinzugefügt werden.

7. Verfahren nach einem der Ansprüche 1 - 6, **dadurch gekennzeichnet, dass** die Substanz aus einer Gruppe ausgewählt ist, welche aus Metaboliten, Tracern für Infektionskrankheiten, toxikologisch relevanten Substanzen und Tracern dafür besteht, wobei die Substanz vorzugsweise Glucose ist.

## Revendications

1. Méthode pour déterminer la fiabilité d'un dispositif de mesure de la concentration d'une substance dans le sang total, comprenant les étapes successives suivantes consistant à :
a) mélanger une fraction de sang séparée, stockée et acellulaire, choisie parmi du plasma et du sérum, et une suspension de cellules sanguines, chacune séparée du sang témoin total, avec une quantité connue de ladite substance étant ajoutée à ladite fraction de sang acellulaire, dans lequel ladite fraction de sang acellulaire et ladite suspension de cellules sanguines sont stockées dans un conteneur qui comprend au moins deux composants connectés, de préférence à une température de 2 à 8°C ;
b) mesurer, au moyen dudit dispositif, la concentration de ladite substance dans le sang témoin total reconstitué enrichi avec ladite substance à l'étape a) ;
c) déterminer la fiabilité dudit dispositif sur la base de la différence entre (i) la concentration connue de ladite substance après l'étape a) et (ii) le résultat de la mesure de l'étape b).

2. Méthode selon la revendication 1, **caractérisée en ce que** ladite fraction de sang acellulaire et ladite suspension de cellules sanguines à utiliser à l'étape (a) sont obtenues en centrifugeant du sang témoin total et en enlevant 5 à 90 % en volume de la fraction de sang acellulaire de manière à obtenir une suspension de cellules sanguines, facultativement en utilisant un ou plusieurs additifs, dans laquelle de préférence 75 à 85 % en volume de la fraction de sang acellulaire sont enlevés.

3. Méthode selon la revendication 2, **caractérisée en ce que** la teneur en hématocrite de la suspension de cellules sanguines est ajustée entre 20 et 90, dans laquelle de préférence la teneur en hématocrite est ajustée entre 50 et 80 % en volume.

4. Méthode selon l'une quelconque des revendications 1 à 3, **caractérisé en ce que** ledit sang témoin total est constitué de sang de mammifère, y compris du sang humain, de préférence ledit sang est du sang bovin ou du sang humain.

5. Méthode selon l'une quelconque des revendications 1 à 4, **caractérisée en ce que** ladite fraction de sang acellulaire enrichie en substance est stockée de manière aseptique dans des flacons stériles.

6. Méthode selon l'une quelconque des revendications 1 à 5, **caractérisée en ce que** ladite substance est ajoutée à ladite fraction de sang acellulaire en une quantité telle qu'une concentration souhaitée de ladite substance dans un échantillon soit obtenue après reconstitution de ladite fraction de sang acellulaire avec une quantité de ladite suspension de cellules sanguines dans un échantillon de sang témoin total enrichi avec ladite substance, dans laquelle de préférence des concentrations différentes des substances sont ajoutées à différents échantillons de ladite fraction de sang acellulaire.

7. Méthode selon l'une quelconque des revendications 1 à 6, **caractérisé en ce que** ladite substance est choisie dans un groupe constitué de métabolites, de traceurs pour des maladies infectieuses, de substances pertinentes sur le plan toxicologique et de traceurs pour substances pertinentes sur le plan toxicologique, de préférence ladite substance est le glucose.
